(19) Europäisches Patentamt

European Patent Office

Office européen des brevets

(11) Publication number: **0 254 811 B1**

(12) **EUROPEAN PATENT SPECIFICATION**

(45) Date of publication of patent specification: **18.08.93**   (51) Int. Cl.⁵: **B01J 39/04**, A61K 9/18

(21) Application number: **87105503.4**

(22) Date of filing: **14.04.87**

(54) **Coated ion exchange resins.**

(30) Priority: **30.07.86 US 892169**

(43) Date of publication of application:
**03.02.88 Bulletin 88/05**

(45) Publication of the grant of the patent:
**18.08.93 Bulletin 93/33**

(84) Designated Contracting States:
**AT BE CH DE ES FR GB GR IT LI LU NL SE**

(56) References cited:
**EP-A- 0 171 528
GB-A- 982 150
US-A- 3 594 470
US-A- 4 221 778**

(73) Proprietor: **FISONS CORPORATION
Jefferson Road
Rochester, NY 14603(US)**

(72) Inventor: **Raghunathan, Yegnaswami
6 Dunham Circle
Fairport New York 14450(US)**
Inventor: **Chow, San-Laung
4 Shagbark Way
Fairport New York 14450(US)**

(74) Representative: **Wright, Robert Gordon McRae
FISONS plc 12 Derby Road
Loughborough Leicestershire LE11 0BB (GB)**

EP 0 254 811 B1

**Description**

The present invention relates to sulfonic acid cationic exchange resin particles which have been treated with a small amount of certain, enumerated impregnating agents to improve the particles coatability, and to selective, prolonged continuous release pharmaceutical preparations made therefrom. As described in U.S. Patent No. 4,221,778, the contents of which are incorporated herein by reference, such preparations contain ion exchange resin particles having a pharmacologically active drug adsorbed thereon to form drug-resin complex particles, at least a portion of which particles are treated with an impregnating agent and provided with a diffusion barrier coating.

BACKGROUND

U.S. Patent No. 4,221,778 discloses that controlled (i.e., selective, prolonged) continuous release of pharmacologically active drugs, under conditions such as those encountered in the gastrointestinal tract, can be achieved by the application of diffusion barrier coatings to ion exchange resin drug complex particles which have been treated, prior to coating, with up to about 30% by weight of an impregnating agent selected from polyethylene glycol, propylene glycol, mannitol, lactose, and methylcellulose. The examples of the patent show that the impregnating agent is normally used at a level of about 25% by weight.

BRIEF SUMMARY OF THE INVENTION

The present invention is concerned (a) with sulfonic acid cationic exchange resin particles which have been treated to improve their coatability with up to about 20 percent by weight (based on the combined weight of the particles and the treating agent) of an effective amount of an impregnating agent selected from hydroxypropylmethyl cellulose, hydroxypropyl cellulose, sorbitol, hydroxypropyl sorbitol, and polyvinylpyrrolidone which have a basic drug adsorbed thereon to form drug-resin complex particles, (b) with such drug-resin complex particles which have been coated, subsequent to treatment with the impregnating agent, with a water-permeable diffusion barrier, and (c) with pharmaceutical preparations comprised of such coated drug-resin complex particles. Prolonged continuous release of the drug is obtainable from such preparations under conditions encountered in the gastrointestinal tract.

By varying the amount of coating, and/or by blending coated drug-resin complex particles with uncoated drug-resin complex particles, it is possible to selectively modify the preparation's drug dissolution profile as desired.

The ion exchange resins, drugs, and coatings, and methods for preparing drug-resin complexes, for coating of the complexes, and for selectively modifying the preparation's dissolution profile through blending and/or degree of coating, are disclosed and exemplified in U.S. Patent No. 4,221,778, said disclosure and examples being herein incorporated by reference.

DETAILED DESCRIPTION

It has now been found that the aforementioned treating agents of this invention, while not useful at the higher levels disclosed in U.S. 4,221,778, are effective in amounts of up to about 20 percent by weight, based on the weight of the resin and the treating agent (equivalent to about 15 percent by weight based on the weight of the drug-resin complex and the treating agent). The hydroxypropylmethyl cellulose and the hydroxypropyl cellulose are used at a level of from about 3 to about 20% by weight (preferably 7 to 13%); below 3% is workable but impractical since the impregnating solution becomes too thin, while above 20% the impregnating solution becomes too thick and viscous. The sorbitol and hydroxypropyl sorbitol are used at a level of from about 7 to about 20% by weight (preferably 7 to 13%); above or below the 7-20% range these agents begin to lose their effectiveness. The polyvinylpyrrolidone is used at a level of from about 7 to about 20% by weight (preferably 13 to 20%); below 7% it is less effective and above about 20% it becomes too viscous. All of the foregoing percentages are based on the combined weight of the resin and the impregnating agent. As shown in Table I and II hereinafter, treatment of the resin with the agents of this invention (Examples 1 to 5), in comparison to untreated resin (Comparative Examples A and B), enhanced the resin's coatability as evidenced by the greater retardation in the drug release rate in a simulated gastrointestinal fluid (0.1 normal hydrochloric acid) for the coated drug-resin complexes made from the treated resin.

In general, all pharmacologically active basic drugs, especially those having short biological half-lives in the order of up to about eight hours, are potential candidates for inclusion in the subject preparations. Examples include, but are not limited to, phenylpropanolamine (PPA), dextromethorphan, codeine, hydrocodone, hydralazine, propranolol, doxepin, metaproterenol, morphine, ephedrine, pseudophedrine, and verapamil. PPA, a sympathomimetic amine drug with a biological half life of 3.9 hours in man and a pKa of 9.4, was chosen as a model drug for use in the illustrative examples. The loading of the drug on the resin particles can be from about 1 to about 90 percent by weight, although 15 to 50 percent is the normal practical range.

The resin is a sulfonic acid cationic exchange resin, normally in particle sizes ranging from about 25 to about 1000 $\mu$m. The illustrative examples employ Amberlite® IRP-70 resin, a cationic exchange resin which is 100-200 mesh (75-150 $\mu$m) fractured resin particles of Amberlite IR-120. The parent resin of Amberlite IR-120 and Amberlite IRP-70 is described by the manufacturer as a gel-type divinylbenzene sulfonic acid cationic exchange resin which swells in water with a pH range of 0-14. The resin should not have inherent pharmacological or toxic properties.

Adsorption of the drug onto the ion exchange resin particles to form the drug resin complex is a well known technique as shown in U.S. Patent Nos. 2,990,332 and 4,221,778. In general the drug is mixed with an aqueous suspension of the resin, and the complex is then washed and dried. Adsorption of drug onto the resin may be detected by a change in the pH of the reaction medium.

The water-permeable, diffusion barrier coating material can in general be any of the conventional synthetic or natural film-forming materials with diffusion barrier properties and with no inherent pharmacological or toxic properties. Ethylcellulose, a water insoluble film-forming agent was used as the model diffusion barrier membrane material in the illustrative examples. A plasticizer, Durkex® 500 vegetable oil, was used to improve the film-forming characteristics of ethylcellulose. The amount of coating used depends on the degree of drug release prolongation desired.

Conventional coating solvents (such as ethanol, or a methylene chloride/acetone mixture, or coating emulsions) and coating procedures (such as fluid bed coating) can be employed to coat the particles. Techniques of fluid bed coating are taught, for example, in U.S. Patent Nos. 3,089,824; 3,117,027; and 3,253,944. The coating is normally applied to the drug resin complex, but alternatively can be applied to the resin before complexing with the drug.

In the examples to follow the drug-resin complex particles are coated at a level of 16 percent by weight (as dry weight of the non-volatile solids of the coating, complex, and treating agent, if any) with a coating solution containing about 5% by weight ethylcellulose (50cps), about 2% by weight Durkex 500 refined vegetable oil, about 10% by weight acetone, and the balance methylene chloride. The coating solution is prepared by dissolving the Durkex and ethylcellulose in the acetone and methylene chloride. The coating of the complex particles is carried out in a fluid bed coating apparatus at a rate of 16-17 ml of coating solution per minute. The inlet air temperature is about 33°C and the outlet air temperature is about 22-25°C.

While the impregnating agent is normally applied to the complex, it may be applied to the resin particles prior to complexing, as in the case where the resin particles are coated prior to complexing with the drug. In the following examples the impregnating agent, dissolved in sufficient deionized water, is added to the PPA-resin complex and mixed (in a suitable planetary mixer) for about 10 minutes, then fluid bed dried to a moisture of less than 5%.

Variation in the amount of coating and/or the use of coated/uncoated complex mixtures can be employed to selectively modify the dissolution profile as desired. In addition to oral administration, the preparations of the subject invention are also suitable for topical, rectal or vaginal administration in dosages varying over a wide range, for example, from about 0.1 to about 1000 mg, depending on the nature of the drug and its intended usage. The compositions can take the form of tablets, powders, capsules, liquid suspensions or other conventional dosage forms.

The following dissolution test apparatus and procedures are used in the examples to simulate conditons encountered in the gastrointestinal tract: Five hundred ml of the dissolution medium (0.1N HCl) is placed in a round bottom flask immersed in a suitable water bath and the temperature allowed to rise to 37±0.5°C. The flask is equipped with a paddle which is agitated at 100 rpm. The dissolution medium is pumped from the vessel through a cotton filter. Polyethylene tubing carries the filtered media via a peristaltic pump through a 1 cm flow cell of a Beckman model 35 recording spectrophotometer (equipped with a cell changer) and returns it to the vessel. The flow rate is adjusted to 16 ml/minute. In this way, each of the six vessels and a standard can be monitored at 15 minutes or other suitable intervals. The spectrophotometer is operated at 257 nm in a single beam mode to monitor six resin complex samples and one PPA hydrochloride standard. Each dissolution vessel contains resin complex sample equivalent to 90.6 mg of PPA base. The standard PPA solution contains 90.6 mg of PPA based in 500 ml of 0.1 N HCl. The drug released is then expressed as a percentage of the total drug present in the complex particles.

ILLUSTRATIVE EXAMPLES

Control - Uncoated, Untreated Complex:

PPA hydrochloride (96.84 kg) was dissolved in 850 liters of deionized water in a glass-lined mixing kettle. Amberlite IRP-70 resin (243.77 kg), washed hydrogen cycle, was then added to the stirring PPA hydrochloride solution. The mixing was continued for 2 hours. The PPA-resin slurry was then transferred to the holding tank and then to the centrifuge. The resin core was washed with deionized water for at least 10 minutes until free of chloride ions. The resin core was then dried to a moisture of less than 5% in a fluid bed dryer having a 70°C inlet air temperature. The dried resin complex was found to contain 23.8% of phenylpropanolamine. Dissolution results are shown in Table I.

Comparative Example A - Coated, Untreated, Complex:

Dissolution results on the PPA-resin complex particles, coated as described hereinabove, are shown in Table I.

Examples 1 to 4 - Pretreated, Coated Complex:

Four batches of coated PPA-resin complex particles were prepared, one of which was pretreated with about 13% hydroxypropylmethyl cellulose (Example 1), one with about 13% hydroxypropyl cellulose (Example 2), one with about 13% sorbitol (Example 3), and one with about 13% hydroxypropyl sorbitol (Example 4), all percents being by weight based on the weight of the resin and the impregnating agent. The dissolution results are shown in Table I.

## Table I

### % PPA Released At:

|  | 0.25 hr. | 0.5 hr | 1.0 hr. | 3.0 hr. |
|---|---|---|---|---|
| Control | 86.4 | 86.5 | 86.4 | 93.0 |
| Comparative Example A | 21.0 | 30.9 | 41.7 | 58.1 |
| Example 1 | 13.9 | 20.6 | 29.9 | 47.4 |
| Example 2 | 14.7 | 20.2 | 29.0 | 41.6 |
| Example 3 | 20.8 | 23.8 | 28.2 | 40.3 |
| Example 4 | 14.8 | 21.2 | 31.3 | 52.4 |

Comparative Example B - Coated, Untreated Complex

Dissolution results on coated, unimpregnated PPA-resin complex, made using a second batch of resinated complex and coated as described hereinabove, are shown in Table II.

Example 5 - Pretreated, Coated Complex

PPA-resin complex from the second batch, pretreated with about 20% polyvinylpyrrolidone, was coated as described hereinabove. The dissolution results are shown in Table II.

## Table II

### % PPA Released At:

| | 0.25 hr. | 0.5 hr | 1.0 hr. | 3.0 hr. |
|---|---|---|---|---|
| Comparative Example B | 39.4 | 51.9 | 76.6 | 84.3 |
| Example 5 | 22.0 | 28.8 | 37.8 | 56.5 |

Applying the foregoing discovery, controlled release dosage forms can be formulated for human or veterinary use to contain suitable mixtures of coated and uncoated complex particles such that a desired controlled release profile of the drug is obtained. The dosage forms can be solid (such as powders, capsules and tablets) or liquid (such as a suspension of complex particles in a palatable vehicle).

**Claims**
**Claims for the following Contracting States : BE, CH, DE, FR, GB, IT, LI, LU, NL, SE**

1. Drug resin complex particles comprising sulphonic acid cationic exchange resin particles treated with an impregnating agent, and a basic drug absorbed thereon, characterised in that the particles have been treated with from 3 to 20 percent by weight of the agent, based on the combined weight of the agent and the resin particles, the agent being selected from the group consisting of hydroxypropylmethyl cellulose, hydroxypropyl cellulose, sorbitol, hydroxypropyl sorbitol and polyvinyl pyrrolidone.

2. Drug-resin complex particles as in Claim 1 which have been coated, subsequent to treatment with the impregnating agent, with a water-permeable diffusion barrier.

3. Pharmaceutical preparations comprised of the coated drug-resin complex particles of Claim 2.

4. The preparation of Claim 3 wherein the impregnating agent is hydroxypropylmethyl cellulose.

5. The preparation of Claim 3 wherein the impregnating agent is hydroxypropyl cellulose.

6. The preparation of Claim 3 wherein the impregnating agent is sorbitol.

7. The preparation of Claim 3 wherein the impregnating agent is hydroxypropyl sorbitol.

8. The preparation of Claim 3 wherein the impregnating agent is polyvinylpyrrolidone.

**Claims for the following Contracting States : AT, ES, GR**

1. A process for the preparation of drug resin complex particles comprising sulphonic acid cationic exchange resin particles treated with an impregnating agent, and a basic drug absorbed thereon, characterised in that the particles are treated with from 3 to 20 percent by weight of the agent, based on the combined weight of the agent and the resin particles, the agent being selected from the group consisting of hydroxypropylmethyl cellulose, hydroxypropyl cellulose, sorbitol, hydroxypropyl sorbitol and polyvinyl pyrrolidone.

2. The process of Claim 1, characterized in that the drug-resin complex particles have been coated with a water-permeable diffusion barrier subsequent to treatment with the impregnating agent.

3. The process of Claims 1 and 2, wherein the impregnating agent is hydroxypropylmethyl cellulose.

4. The process of Claims 1 and 2, wherein the impregnating agent is hydroxypropyl cellulose.

5. The process of Claims 1 and 2, wherein the impregnating agent is sorbitol.

6. The process of Claims 1 and 2, wherein the impregnating agent is hydroxypropyl sorbitol.

7. The process of Claims 1 and 2, wherein the impregnating agent is polyvinylpyrrolidone.

**Patentansprüche**
**Patentansprüche für folgende Vertragsstaaten : BE, CH, DE, FR, GB, IT, LI, LU, NL, SE**

1. Arzneimittel-Harzkomplexteilchen umfassend mit einem Imprägniermittel behandelte Sulfonsäurekationenaustauscherharzteilchen und ein darauf absorbiertes Grundarzneimittel, dadurch gekennzeichnet, daß die Teilchen mit 3 bis 20 Gew.% des Mittels, bezogen auf das Kombinationsgewicht von Mittel und Harzteilchen, behandelt wurden, wobei das Mittel ausgewählt ist aus der Gruppe bestehend aus Hydroxypropylmethylzellulose, Hydroxypropylzellulose, Sorbit, Hydroxypropylsorbit und Polyvinylpyrrolidon.

2. Arzneimittel-Harzkomplexteilchen nach Anspruch 1, die nach der Behandlung mit dem Imprägniermittel mit einer wasserdurchlässigen Diffusionssperre beschichtet wurden.

3. Pharmazeutische Präparate bestehend aus den beschichteten Arzneimittel-Harzkomplexteilchen nach Anspruch 2.

4. Präparat nach Anspruch 3, in dem das Imprägniermittel Hydroxypropylmethylzellulose ist.

5. Präparat nach Anspruch 3, in dem das Imprägniermittel Hydroxypropylzellulose ist.

6. Präparat nach Anspruch 3, in dem das Imprägniermittel Sorbit ist.

7. Präparat nach Anspruch 3, in dem das Imprägniermittel Hydroxypropylsorbit ist.

8. Präparat nach Anspruch 3, in dem das Imprägniermittel Polyvinylpyrrolidon ist.

**Patentansprüche für folgende Vertragsstaaten : AT, ES, GR**

1. Verfahren zur Herstellung von Arzneimittel-Harzkomplexteilchen umfassend mit einem Imprägniermittel behandelte Sulfonsäurekationenaustauscherharzteilchen und ein darauf absorbiertes Grundarzneimittel, dadurch gekennzeichnet, daß die Teilchen mit 3 bis 20 Gew.% des Mittels, bezogen auf das Kombinationsgewicht von Mittel und Harzteilchen, behandelt werden, wobei das Mittel ausgewählt ist aus der Gruppe bestehend aus Hydroxypropylmethylzellulose, Hydroxypropylzellulose, Sorbit, Hydroxypropylsorbit und Polyvinylpyrrolidon.

2. Verfahren nach Anspruch 1, dadurch gekennzeichnet, daß die Arzneimittel-Harzkomplexteilchen nach der Behandlung mit dem Imprägniermittel mit einer wasserdurchlässigen Diffusionssperre beschichtet wurden.

3. Verfahren nach den Ansprüchen 1 und 2, in dem das Imprägniermittel Hydroxypropylmethylzellulose ist.

4. Verfahren nach den Ansprüchen 1 und 2, in dem das Imprägniermittel Hydroxypropylzellulose ist.

5. Verfahren nach den Ansprüchen 1 und 2, in dem das Imprägniermittel Sorbit ist.

6. Verfahren nach den Ansprüchen 1 und 2, in dem das Imprägniermittel Hydroxypropylsorbit ist.

7. Verfahren nach den Ansprüchen 1 und 2, in dem das Imprägniermittel Polyvinylpyrrolidon ist.

**Revendications**
**Revendications pour les Etats contractants suivants : BE, CH, DE, FR, GB, IT, LI, LU, NL, SE**

1. Particules de complexe médicament-résine comprenant des particules de résine échangeuse à cations d'acide sulfonique traitées avec un agent d'imprégnation et un médicament basique adsorbé sur elles, caractérisées en ce que les particules ont été traitées avec 3 à 20% en poids de l'agent, sur base du poids combiné de l'agent et des particules de résine, l'agent étant choisi dans le groupe formé par l'hydroxypropylméthylcellulose, l'hydroxypropylcellulose, le sorbitol, l'hydroxypropylsorbitol et la polyvinylpyrrolidone.

2. Particules de complexe médicament-résine selon la revendication 1, qui ont été enrobées, après le traitement avec l'agent d'imprégnation, avec une barrière de diffusion perméable à l'eau.

3. Compositions pharmaceutiques formées des particules de complexe médicament-résine enrobées de la revendication 2.

4. Composition selon la revendication 3, dans laquelle l'agent d'imprégnation est l'hydroxypropylméthylcellulose.

5. Composition selon la revendication 3, dans laquelle l'agent d'imprégnation est l'hydroxypropylcellulose.

6. Composition selon la revendication 3, dans laquelle l'agent d'imprégnation est le sorbitol.

7. Composition selon la revendication 3, dans laquelle l'agent d'imprégnation est l'hydroxypropylsorbitol.

8. Composition selon la revendication 3, dans laquelle l'agent d'imprégnation est la polyvinylpyrrolidone.

**Revendications pour les Etats contractants suivants : AT, ES, GR**

1. Procédé de préparation de particules de complexe médicament-résine comprenant des particules de résine échangeuse à cations d'acide sulfonique traitées avec un agent d'imprégnation et un médicament basique adsorbé sur elles, caractérisé en ce que les particules sont traitées avec 3 à 20% en poids de l'agent, sur base du poids combiné de l'agent et des particules de résine, l'agent étant choisi dans le groupe formé par l'hydroxypropylméthylcellulose, l'hydroxypropylcellulose, le sorbitol, l'hydroxypropylsorbitol et la polyvinylpyrrolidone.

2. Procédé de la revendication 1, caractérisé en ce que les particules de complexe médicament-résine ont été enrobées avec une barrière de diffusion perméable à l'eau, après le traitement avec l'agent d'imprégnation.

3. Procédé selon les revendications 1 et 2, dans lequel l'agent d'imprégnation est l'hydroxypropylméthylcellulose.

4. Procédé selon les revendications 1 et 2, dans lequel l'agent d'imprégnation est l'hydroxypropylcellulose.

5. Procédé selon les revendications 1 et 2, dans lequel l'agent d'imprégnation est le sorbitol.

6. Procédé selon les revendications 1 et 2, dans lequel l'agent d'imprégnation est l'hydroxypropylsorbitol.

7. Procédé selon les revendications 1 et 2, dans lequel l'agent d'imprégnation est la polyvinylpyrrolidone.